# EUROPÄISCHE PATENTSCHRIFT

(11) **EP 2 132 383 B1**
(45) Veröffentlichungstag und Bekanntmachung des Hinweises auf die Patenterteilung: **17.04.2013**
(21) Anmeldenummer: 07847219.8
(22) Anmeldetag: 20.11.2007
(51) Int. Cl.: E03D 9/00, E03D 9/03

(54) **System mit einer Nachfülleinheit mit einer lösbaren Funktionskammer mit Sprühkopf**
System having a refilling unit having a removable functional chamber with a spray head
Système comprenant une unité de recharge comprenant une chambre fonctionnelle amovible avec un aérosol

(30) Priorität: 09.03.2007 DE 102007011991
(43) Veröffentlichungstag der Anmeldung: 16.12.2009
(60) Teilanmeldung: 10181965.4
(73) Patentinhaber: Henkel Kommanditgesellschaft auf Aktien, 40589 Düsseldorf (DE)
(72) Erfinder: PESSEL, Frank, 40215 Düsseldorf (DE); MÜHLHAUSEN, Hans-Georg, 40597 Düsseldorf (DE); BUTTER-JENTSCH, Ralph, 40764 Langenfeld (DE)
(86) Internationale Anmeldenummer: PCT/EP2007/062531
(87) Internationale Veröffentlichungsnummer: WO 2008/110221

(56) Entgegenhaltungen:
- EP-A- 1 334 239
- EP-A- 1 460 189
- EP-A- 1 522 319
- WO-A-03/066979
- WO-A-2006/005410
- GB-A- 2 327 884
- GB-A- 2 372 046
- GB-A- 2 423 531
- US-A- 4 212 089

## Beschreibung

Die Erfindung betrifft ein System gemäß dem Oberbegriff von Anspruch 1 mit einer Nachfülleinhet mit einer lösbaren Funktionskammer für ein an den Rand eines Toilettenbeckens fixierbaren Wirkstoffabgabesystems, das umgangssprachlich auch als WC-Körbchen bekannt ist.

### Stand der Technik

Aus dem Stand der Technik ist eine Vielzahl von unterschiedlichen Wirkstoffabgabesystemen für Toilettenbecken bekannt.

Es existieren Wirkstoffabgabesysteme für Wirkstoffzubereitungen, bei denen die Wirkstoffe aus einer festen Matrix herauslösbar sind. Derartige, feste Wirkstoffzubereitungen werden auch als sog. Rim-Blocks bezeichnet. Bekannt sind ferner Wirkstoffzubereitungen in gelförmiger und flüssiger Form.

Allen dieser Wirkstoffzubereitungen ist üblicherweise gemein, dass sie einen oder mehrere Duftstoffe beinhalten.

Bekannt sind zunächst Abgabevorrichtungen für eine einzelne Wirkstoffzubereitung. Die Zubereitung befindet sich üblicherweise in einem Halter fest angeordneten oder auswechselbar eingesetzten Vorratsbehälter. Mit einem am Halter ausgebildeten Bügel wird das Wirkstoffabgabesystem am Rand der Toilette befestigt, wobei der Vorratsbehälter oder dafür vorgesehene Abgabeeinrichtungen, beispielsweise Abspülplatten, auf der Toiletteninnenseite unmittelbar unterhalb des Toilettenrandes anliegen, so dass beim Spülwasseraustritt aus dem innenseitigen Toilettenrand der Vorratsbehälter oder die entsprechenden Abgabevorrichtungen vom Spülwasser überströmt werden. Derartige Abgabevorrichtungen sind beispielsweise aus EP1334239, WO03/066979A1 oder WO02/40791A1 bekannt.

Ein wesentlicher Nachteil derartiger Wirkstoffabgabesysteme ist es, dass eine Beduftung des Toilettenbeckens bzw. der Umgebung des Toilettenbeckens im Wesentlichen an den Spülvorgang der Toilette gekoppelt ist, da derartige Wirkstoffabgabesysteme so ausgebildet sind, dass üblicherweise nur dann ein Parfum enthaltener Wirkstoff aus den eingangs genannten Wirkstoffabgabesystemen freigesetzt wird, wenn das Wirkstoffabgabesystem von Spülwasser überströmt wird.

Eine andere Lösung offenbart GB2327884A oder auch US4670916. welche beide jeweils eine Duftabgabevorrichtung beschreiben, die durch das Aufsitzen eines Benutzers auf die Tollettenbrille ausgelöst werden und so eine intervallartige Duftstofffreisetzung in das Toilettenbecken bewirken.

Eine intervallartige Duftslofffreisetzung, die an das Befüllen und Entleeren des Spülwassertanks eines WCs gekoppell ist wird in US4212089 offenbart.

Bei all den vorgenannten Systemen ist der Dufteindruck intervallartig und regelmäßig von nur kurzer Dauer. Insbesondere wird keine Beduftung vor Benutzung einer Toilette durch die Wirkstoffabgabesysteme der eingangs geschilderten Art bewirkt.

Dieser Nachteil führte zur Entwicklung von WC-Abgabesystemen der eingangs geschilderten Art, die eine permanente Abgabe eines Duftstoffs in die Umgebung eines Toilettenbeckens erlauben.

Aus EP1460189A1 ist beispielsweise ein WC-Körbchen für flüssige Wirkstoffzubereitungen bekannt, der eine in der Nachfülleinheit integrierte Parfumkammer aufweist. Ein ähnliches WC-Körbchen wird auch in GB2372046A, GB2423531A oder EP1522319A1 offenbart

Aus WO2005/093176 ist ferner ein WC-Körbchen bekannt, In dessen Gehäuse ein gelförmiger. Duftstoff enthaltener Block fest eingegossen ist. Nachteilig an dieser Lösung ist, dass der gelförmige Block derart angeordnet ist, dass er von Spülwasser überströmt und somit allmalig aufgelöst wird, wodurch nach und nach ein äußerst unanehmlicher optischer Eindruck entsteht.

Dieses Problem wird beispielsweise durch ein WC-Wirksloffabgabesystem gemäß WO2006/005410 vermieden. Bei dem aus der vorgenannten Offenbarung bekannten WC-Korbchen, sind Duftstoff enthaltende Kammern am Halter der Abgabevorrichtung angeordnet. Durch das Einschließen der Duftstoffzubereitung in einer Kammer, wird eine unbeabsichtigte Freisetzung der Zubereitung durch spülwasserbedingte Auflösung verhindert. Ferner wird es durch die Verwendung einer Kammer möglich, Flüssigkeiten, welche mit höheren Konzentrationen an Duftstoff beladbar sind, als Duftstoffträger einzusetzen.

Ein wesentlicher Nachteil dieser Lösung ist es Jedoch, dass die Duftstoffkammer fest am Halter angeordnet ist. Üblicherweise werden nämlich bei derartigen Wirkstoffabgabesystemen entieerte Vorratsbehälter in Form von Nachfülleinheiten durch neue, gefüllte Vorratsbehälter ausgetauscht. Der Halter verbleibt dabei regelmäßig am Toilettenrand. Bei dem Wirkstoffabgabesystem der o.g. Art ist es daher notwendig, entweder den Halter nach vollständiger Abgabe der Duftstoffe aus der Duftstoffkammer zu ersetzen oder aber die Duftstoffkammer vom Halter zu entfernen und gegen eine neue auszutauschen. Melde Varianten sind nachteilig, da zum Einen der Halter mit der verbrauchten Duftstoffkammer üblicherweise noch funktionsfähig ist und daher eigentlich keines Austausch bedarf und zum Anderen da beim Austausch der verbrauchten Duftstoffabgabekammer ein zusätzlicher Handhabungsschritt des Benutzers unmittelbar an einer regelmäßig als äußerst unhygienisch empfunden, da unmittelbar im Spülwasserstrom befindlichen, Anordnung- nämlich dem Halter- notwendig ist.

Da die aus WO2006/005410 bekannte Duftstoffkammer unmittelbar an dem Halter des Wirkstoffabgabesystems fixiert wird, ragt die Duftstoffkammer Ober den Rand eines üblichen Halters hinaus in das Toilettenbecken hinein. Da ein WC-Wirkstoffabgabesystem die benutzbare Toilettenbeckenöffnung so wenig wie möglich beschränken soll, wird eine derartige Konstruktion von Benutzern jedoch häufig als störend empfunden. So ist eine vom Anwender häufig gewünschte, diskrete Anordnung eines derartigen WC-Spülers unter dem Rand einer Toilette üblicherweise nicht möglich.

### Aufgabe der Erfindung

Aufgabe der Erfindung ist es daher, die aus dem Stand der Technik bekannten Nachteile zu überwinden und ein WC-Abgabesystem der eingangs bezeichneten Art bereitzustellen, dass eine permanente und benutzerbewirkte Beduftung der Umgebung eines Toilettenbeckens realisiert ohne den Halter des WC-Abgabesystems austauschen zu müssen.

Eine weitere Aufgabe der Erfindung ist es, dass die Duftstoffkammer auf einfache und hygienische Art und Weise in dem Duftstoffabgabesystem austauschbar Ist.

Ferner liegt der Erfindung die Aufgabe zugrunde, ein WC-Abgabesystem zu schaffen, dass so gering wie möglich in das Innere des Toilettenbeckens hineinragt.

Diese Aufgabe wird durch ein System zum Einbringen wenigstens einer Wirkstoffsubstanz enthaltenen Zubereitung in das Spülwasser eines Tollettenbeckens mit den Merkmalen des Anspruchs 1 gelöst.

Im Sinne dieser Anmeldung sind durch den Begriff Toilettenbecken, Wasserklosetts, Urinale, Bidets und dergleichen mit umfasst.

### Funktionskammer

Eine Funktionskammer im Sinne dieser Anmeldung bezeichnet einen von der Nachfülleinheit eines WC-Körbchens separierbaren Raum, der dazu geeignet ist, eine Vorrichtung und/oder Zubereitung zumindest teilweise zu umhüllen und/oder zusammenzuhalten.

Die Funktionskammer kann insbesondere als jegliche Art von Behälter, Ampulle, Dose, Aerosoldose, Beutel, Flasche, Kartusche, Kapsel, Aufwickelhülse, Rollenkern, Ballon, Flakon, Röhrchen, Tiegel, Kammer, Blister, Korb oder Geflecht ausgebildet sein. Im Sinne dieser Anmeldung umfasst die Funktionskammer auch einen aus einer festen oder gelförmigen Zubereitung geformten Körper.

Die Funktionskammer kann aus einem oder mehreren voneinander getrennten oder beanstandeten Räumen bestehen.

Die Funktionskammer ist lösbar an der Nachfülleinheit fixiert, insbesondere durch Form-, Kraft,- und/oder Stoffschluss.

Die Funktionskammer und die Nachfülleinheit können insbesondere so ausgebildet sein und/oder zusätzliche Verbindungsmittel umfassen, so dass die Funktionskammer und die Nachfülleinheit formschlüssig beispielsweise durch einen Schnappverschluss, Rastverschluss, Prellverschluss, Schraubverschluss, Bajonettverschluss, Klemmverschluss, Quetschverschluss oder Druckknopfverschluss aneinander lösbar fixierbar sind.

In einer weiteren Ausgestaltungsform der Erfindung kann die Funktionskammer auch stoffschlüssig an der Nachfülleinheit angeordnet sein. Die stoffschlüssige Verbindung ist insbesondere in derart ausgebildet, dass ein wiederholtes Lösen und Haften der Funktionskammer auf bzw. an der Nachfülleinheit ermöglicht wird. Die stoffschlüssige Verbindung kann beispielsweise aus der Gruppe der Klebeverbindungen, Schweißverbindungen oder Siegelverbindungen ausgewählt sein.

Ferner ist es möglich die lösbare Verbindung zwischen der Funktionskammer und der Nachfülleinheit durch Kraftschluss herzustellen. Dies kann beispielsweise durch eine Pressverbindung, Reibverbindung, Klemmverbindung, Quetschverbindung, Schrumpfverbindung, Keilverbindung, Schraubverbindung, Stiftverbindung oder dergleichen realisiert sein.

Selbstverständlich kann die lösbare Verbindung zwischen der Funktionskammer und der Nachfülleinheit auch durch eine beliebige Kombination der vorgenannten Verbindungsarten ausgebildet sein.

Gemäß einer bevorzugten Ausführungsform der Erfindung ist die Verbindung zwischen der Funktionskammer und der Nachfülleinheit derart ausgestaltet, dass sie durch eine Zugkraft von größer als 1 N von der Nachfülleinheit lösbar ist. Dies gewährleistet einen hinreichenden Halt der Funktionskammer auf oder in der Nachfülleinheit und verhindert ein unbeabsichtigtes Lösen der Funktionskammer z.B. während des Transports oder Befestigen des WC-Spülers am Rand des Toilettenbeckens.

Bevorzugter Weise weist die Funktionskammer ein Volumen auf zwischen 5 ml und 100 ml, insbesondere zwischen 10 ml und 40 ml, auf.

Ferner kann die Funktionskammer außenseitig ein zusätzliches Befestigungselement aufweisen, das zur permanenten oder temporären Fixierung der Funktionskammer an Strukturen außerhalb des WC-Spülers geeignet ist. Beispielsweise kann das Befestigungselement als versiegelter Klebestreifen ausgebildet sein, wobei die Versiegelung nach Abnahme der Funktionskammer von der Nachfülleinheit abgelöst wird und die Funktionskammer somit beispielsweise auf Oberflächen, insbesondere keramische Oberflächen wie beispielsweise Fliesen oder WC-Becken, in Schränken oder in oder auf Mülleimern aufklebbar ist.

Die Öffnung der Funktionskammer kann in einer weiteren Ausführung der Erfindung mit einer Membran verschlossen sein, die die Trägersubstanz für

Aktivstoffe in der Funktionskammer zurückhält und einen oder mehrere der Aktivstoffe durch die Membran hindurch an die Umgebung abgibt.

In einer weiteren bevorzugten Ausgestaltung der Erfindung weist die Funktionskammer ein Verschlusselement auf, welches in einer Verschlussposition die Abgabe von Aktivsubstanz aus der Funktionskammer an die Umgebung zumindest reduziert und in einer geöffneten Position die Abgabe von Aktivsubstanz aus der Funktionskammer ermöglicht.

Beispielsweise kann das Verschlusselement als Abziehfolie, Abreißdeckel, Aufsatzdeckel, Falzdeckel, Rillendeckel, Scharnierdeckel, Schiebedeckel, Schnappdeckel, Schraubdeckel, Stülpdeckel oder Stopfen ausgeformt sein. Besonders bevorzugt ist es, das Verschlusselement derart auszuführen, dass ein wiederholtes Freigeben und Verschließen der Funktionskammer ermöglicht wird.

Des Weiteren ist es bevorzugt, dass die Funktionskammer zwischen zwei Vorratsbehältern angeordnet ist. Hierdurch ragt die Funktionskammer nicht über Rand des Halters hinaus in das Toilettenbecken hinein und kann gemeinsam mit dem Halter diskret am oder unter den Rand des Toilettenbeckens positioniert werden. Ferner ist durch die Anordnung der Funktionskammer zwischen den Vorratsbehältern ein gewisser Klemmeffekt erreichbar, durch den die Funktionskammer sicher zwischen den Vorratsbehältern fixierbar ist.

Durch die Ausbildung der Funktionskammer als Prismatoid und entsprechend ausgeformten Vorratsbehältern ist diese Klemmwirkung bzw. Fixierung weiter optimierbar. Insbesondere kann die Funktionskammer beispielsweise pyramidenförmig, keilförmig, kuppelförmig, kegelstumpfförmig oder prismenförmig ausgebildet sein.

Durch die entsprechend korrespondierende Formgestaltung der Funktionskammer und Vorratsbehälter wird die Funktionskammer beim Einsetzen durch den Benutzer trichterartig in die in der Nachfülleinheit vorgesehene Position geführt, wodurch Fehlbedienungen beim Einsetzen vermieden werden.

Ausgehend von einer keilförmigen Grundform der Funktionskammer können sich vom Kopf der Funktionskammer flügelartige Abschnitte erstrecken, so dass die Funktionskammer eine im Wesentlichen T-förmige Kontur aufweist. Diese Ausgestaltung ist vorteilhaft, wenn ein größeres Füllvolumen der Funktionskammer benötigt wird.

In einer weiteren Ausgestaltung der Erfindung weist die Funktionskammer einen Docht auf, der aus der Funktionskammer herausragt und durch den mittels Kapillarwirkung, fließfähige Zubereitung aus der Funktionskammer an die Umgebung abgegeben werden kann.

### Handhabungs- und Dosiermittel

An der Funktionskammer sind Handhabungs- und Dosiermittel vorgesehen.

Handhabungsmittel sind an der Funktionskammer ausgebildete Griff- und Trageelemente wie beispielsweise ein Griffstab, Griffloch, Tragebügel, Tragegriff, Fallgriff, eine Griffdaube oder dergleichen.

Die Funktionskammer weist einen Sprühkopf auf, der beim Betätigen des Sprühkopfes die Zubereitung aus der Funktionskammer in den Sprühkopf fördert und durch die Düse des Sprühkopfes in die Umgebung abgibt.

Des Weiteren ist es vorteilhaft, an der Funktionskammer ein Griffelement vorzusehen, durch das der Benutzer die Funktionskammer in der Nachfülleinheit fixiert oder aus ihr entfernt. Das Griffelement ist besonders bevorzugt stielförmig ausgeformt, wodurch ein als teilweise unhygienisch empfundenes Berühren der Funktionskammer vermieden wird. Insbesondere kann das Griffelement als figuratives und/oder florales Element gestaltet sein, beispielsweise als Blüte.

Das Griffelement kann auch mit einem an der Funktionskammer angeordneten Verschlusselement gekoppelt sein, wodurch das Verschlusselement durch das Griffelement bedienbar ist. So kann die Aktivsubstanzfreisetzung durch das Verschlusselement vom Benutzer durch das Griffelement gesteuert werden.

Das Griffelement kann auch zur Steuerung bzw. Bedienung einer in der Funktionskammer befindlichen Vorrichtung ausgebildet sein.

### Zubereitung beinhaltende Funktionskammer

Gemäß einer ersten, bevorzugten Ausführungsform der Erfindung ist in der Funktionskammer eine wenigstens eine Aktivsubstanz beinhaltende Zubereitung angeordnet.

Besonders bevorzugt ist es, dass die Zubereitung(en) in der Funktionskammer von der/den Zubereitung(en) in dem/den Vorratsbehälter(n) verschieden ist/sind.

Die Funktionskammer kann eine oder mehrere Aktivsubstanzen beinhaltende Zubereitungen in flüssiger und/oder gasförmiger Form umfassen.

Gemäß einer zu bevorzugenden Ausführungsform der Erfindung, weist die Zubereitung enthaltende Funktionskammer wenigstens eine Öffnung zur Freisetzung der in ihr bevorrateten Zubereitung auf.

### Mechanische, elektro-mechanische und/oder elektronische Vorrichtung

Die Funktionskammer kann auch eine mechanische, elektro-mechanische, elektro-chemische und/oder elektronische Vorrichtung beinhalten.

Vorzugsweise ist die Funktionskammer zur Aufnahme der Vorrichtung als ein spritzwassergeschütztes Gehäuse, dass das Eindringen von Spritzwasser, wie es beispielsweise bei der Betätigung der WC-Wasserspülung auftreten kann, in das Innere der Funktionskammer verhindert.

Besonders vorteilhaft ist es, die Funktionskammer derart zu vergießen, dass es im Wesentlichen wasserdicht, die Funktionskammer also auch bei vollständigem Umschluss mit Flüssigkeit funktionsfähig ist. Als Vergussmaterialien können beispielsweise mehrkomponentige Epoxyd-, und Acrylat-Vergußmassen wie Methacrylatester, Urethan-Metha und Cyanacrylate oder Zweikomponenten-Materialien mit Polyurethanen, Silikonen, Epoxydharzen verwendet werden.

Gemäß einer bevorzugten Ausführungsform der Erfindung verfügt die Funktionskammer über wenigstens eine Sensoreinheit, die physikalische, chemische und/oder mechanische Parameter aus der Umgebung der Funktionskammer bestimmen kann. Die Sensoreinheit kann einen oder mehrere aktive und/oder passive Sensoren zur qualitativen und/oder quantitativen Erfassung mechanischer, elektrischer, physikalischer und/oder chemischer Größen umfassen, die als Steuersignale verwendet werden.

Insbesondere können die Sensoren aus der Gruppe der Zeitgeber, Infrarotsensoren, Helligkeitssensoren, Temperatursensoren, Bewegungssensoren, Dehnungssensoren, Näherungssensoren, Durchflusssensoren, Farbsensoren, Gassensoren, Vibrationssensoren, Drucksensoren, Leitfähigkeitssensoren, Trübungssensoren, Schallwechseldrucksensoren, "Lab-on-a-Chip"-Sensoren, Kraftsensoren, Beschleunigungssensoren, Neigungssensoren, pH-Wert-Sensoren, Feuchtigkeitssensoren, Magnetfeldsensoren, RFID-Sensoren, Magnetfeldsensoren, Hall-Sensoren, Bio-Chips, Geruchssensoren, Schwefelwasserstoffsensoren und/oder MEMS-Sensoren ausgewählt sein.

Diese eingangs beschriebenen Vorrichtungen können mit einer ebenfalls in der Funktionskammer bevorratenden Zubereitung insbesondere in derart zusammenwirken, dass die Vorrichtung eine sensorbewirkte Aktivsubstanzfreisetzung bewirkt um so beispielsweise eine Luftverbesserung in der Umgebung der Nachfülleinheit zu erzielen.

Die von der Sensoreinheit erzeugten Steuersignale können an eine elektrische oder elektromechanische oder mechanische Steuereinheit weitergeleitet sein, in der die Steuersignale durch in Stellgrößen für einen oder mehrer Aktuatoren umgewandelt und bereit gestellt werden.

Aktuatoren können beispielsweise ausgewählt sein aus der Gruppe der elektrischen, mechanischen, thermischen, chemischen, hydraulischen, pneumatischen Aktuatoren. Insbesondere kann ein Aktuator als Ventil, Pumpe, Ventilator, Motor, Vibrator, Leuchtmittel, Lautsprecher, Bimetall-Aktuator, Piezoelement, Formgedächtniselement oder Heizung ausgebildet sein.

### Vorratsbehälter

Die erfindungsgemäße Nachfülleinheit umfasst wenigstens einen Vorratsbehälter zur Aufnahme einer in das Spülwasser eines Toilettenbeckens abgebbaren Zubereitung.

In einer bevorzugten Ausführungsform der Erfindung ist mindestens ein weiterer Vorratsbehälter vorgesehen, der eine Zubereitung beinhaltet, die von den Zubereitungen der anderen Vorratsbehälter verschieden ist. Dies ist insbesondere vorteilhaft, wenn Zubereitungen verwendet werden sollen, die üblicherweise nicht gemeinsam lagerfähig sind, wie beispielsweise Bleichen und Duftstoffe.

Die Vorratsbehälter können einstückig oder mehrstückig ausgeformt sein. Die einstückige Ausformung von beispielsweise zwei Vorratsbehältern kann durch ein geeignetes Blasformverfahren realisiert sein, wobei die Vorratsbehälter unter Ausbildung eines gemeinsamen Verbindungsstegs einstückig miteinander verbunden sind. Bei einer zweistückigen Ausgestaltung können die beiden voneinander separierbaren Vorratsbehälter beispielsweise über ein entsprechendes Adapterelement gegeneinander fixiert sein.

### Zubereitung

Eine Zubereitung im Sinne dieser Anmeldung ist eine wenigstens eine Aktivsubstanz beinhaltende fließfähige, streufähige, gelförmige, gasförmige oder feste Zusammensetzung.

Durch die Verwendung von fließfähigen Zubereitungen, lassen sich höhere Wirkstoffkonzentrationen und eine verbesserte Freisetzung bzw. Dosierung der Zubereitungen erzielen. Des Weiteren ist es bei der Verwendung von fließfähigen, insbesondere flüssigen Wirkstoffzubereitungen von Vorteil, dass die Vorratsbehälter vor dem Eintritt von Spülwasser geschützt sind, um eine Verdünnung der Zubereitungen zu verhindern.

Die Funktionskammer kann auch eine oder mehrere Zubereitungen in flüssiger und/oder gasförmiger Form beinhalten. Dabei ist es auch denkbar, die entsprechenden Zubereitungen räumlich getrennt voneinander in der Funktionskammer zu bevorraten, um eine ungewollte Vermischung der Zubereitungen zu verhindern.

Eine Zubereitung kann eine oder mehrere gleiche oder unterschiedliche Aktivsubstanzen aus der Gruppe der Duftstoffe, Bleichmittel, Reinigungssubstanzen, Lösemittel, Tenside, Farbstoffe, Enzyme, hygroskopische Substanzen, Flammhemmer, Härter, Verlaufsmittel, Netzmittel, Dispergiermittel, Schaumbildner, Entschäumer, Wachse, Silikone, Entlüfter, Korrosionsschutzmittel, Biozide, Insektizide, Wasserenthärter, Konservierungsmittel, Adsorptionsmittel, Absorptionsmittel, Abrasivstoffe, Emulgatoren, Stabilisatoren, Vitamine, Mineralien und dergleichen umfassen.

### Aktivsubstanzen

Eine Aktivsubstanz im Sinne dieser Anmeldung ist umfasst eine oder mehrere Substanzen aus der Gruppe der Luftverbesserungssubstanzen, Oxidationsmittel, Duftstoffe, Bleichmittel, Reinigungssubstanzen, Lösemittel, Tenside, Farbstoffe, Enzyme, hygroskopische Substanzen, Flammhemmer, Härter, Verlaufsmittel, Netzmittel, Dispergiermittel, Schaumbildner, Entschäumer, Entlüfter, Korrosionsschutzmittel, Biozide, Antimikrobielle Wirkstoffe, Germizide, Fungizide, Antioxidantien, Wasserenthärter, Konservierungsmittel, Emulgatoren, Stabilisatoren, Vitamine, Mineralien und dergleichen.

Die Aktivsubstanzen können auch an oder in Trägermaterialen gebunden oder gelöst sein. Die Trägermaterialien können einen festen und/oder flüssigen und/oder gelartigen und/oder gasförmigen Aggregatszustand aufweisen. Insbesondere kann ein Trägermaterial auch ein poröser Körper sein, wie etwa ein Schwamm. Auch ist es denkbar, dass das Trägermaterial aus Zellulose besteht, wie etwa einem Watte-Pad.

Gemäß einer bevorzugten Ausführungsform der Erfindung, sind die wirkaktiven Substanzen an oder in einem polymeren Trägermaterial gebunden. Besonders bevorzugt werden Duftstoffe in oder an einem polymeren Trägermaterial gebunden.

Weitere Trägermaterialen können beispielsweise auf der Basis von Cellulose, porösen Materialen, wie beispielsweise Schwämmen, abspülbaren oder nicht abspülbaren Gelen, Fluiden, wasserlöslichen Folien oder dergleichen ausgewählt sein.

Die Aktivsubstanzen können des Weiteren auch Substanzen oder Substanzgemische zur Hydrophilisierung von Oberflächen umfassen.

Ferner ist es möglich, Indikatorsubstanzen zur Bestimmung der Wasserhärte, Keimbelastung, Temperatur, Feuchtigkeit usw. in der Kammer zu bevorraten.

Die Aktivsubstanzen können auch optische und/oder akustische Signale erzeugen, beispielsweise durch Fluoreszenz, Phosphoreszenz oder Aufplatzen von zellulären Strukturen. Hierdurch lassen sich beispielsweise optische und/oder akustische Verbrauchsanzeigen realisieren.

Insbesondere kann es nach einer bevorzugten Ausführungsform der Erfindung vorteilhaft sein, eine Abgabe von Aktivstoff in zwei oder mehr Phasen vorzusehen. Dies ist insbesondere dann vorteilhaft, wenn die beiden abzugebenden Phasen nicht gemeinsam lagerstabil sind oder bei der Abgabe von Duftstoff, eine olfaktometrische Anpassung an den abgegebenen Duft vermieden werden soll.

Weitere Ausführungsbeispiele der Erfindung werden anhand der beigefügten Zeichnungen erläutert. Es zeigt:
- Fig. 1: Explosionsansicht des WC-Körbchens mit aus einer Nachfülleinheit entnehmbaren Funktionskammer, das eine nicht beanspruchte Ansführungsform darstellt.
- Fig. 2: Perspektivische Ansicht des WC-Körbchens mit zwei Vorratsbehältern und keilförmiger Funktionskammer, das eine nicht beanspruchte Ausführungsform darstellt.
- Fig. 3: Perspektivische Ansicht des WC-Körbchens mit zwei Vorratsbehältern und keilförmiger Funktionskammer mit Sprühkopf
- Fig. 4: Perspektivische Ansicht des WC-Körbchens mit zwei Vorratsbehältern und T-förmiger Funktionskammer, das eine nicht beanspruchte Ausführungsform darstellt.
- Fig. 5: Perspektivische Ansicht des WC-Körbchens mit einem Vorratsbehälter und Funktionskammer, das eine nicht beanspruchte Ausführungsform darstellt.

### Bezugszeichen

- 1.: WC-Körbchen
- 2.: Halter
- 3.: Adapter
- 4.: Vorratsbehälter
- 5.: Funktionskammer
- 6.: Bügel
- 7.: Öffnungen
- 8.: Öffnungen
- 9.: Folie (Verschlusselement)
- 10.: Griffelement
- 11.: Sprühkopf
- 12.: Nachfülleinheit
- 13.: Auslassöffnung
- 14.: Zwischenraum
- 15.: Außenwand
- 16.: Außenwand
- 17.: Bügel
- 18.: figuratives Element
- 19.: Funktionskammerabschnitt
- 20.: Funktionskammerabschnitt

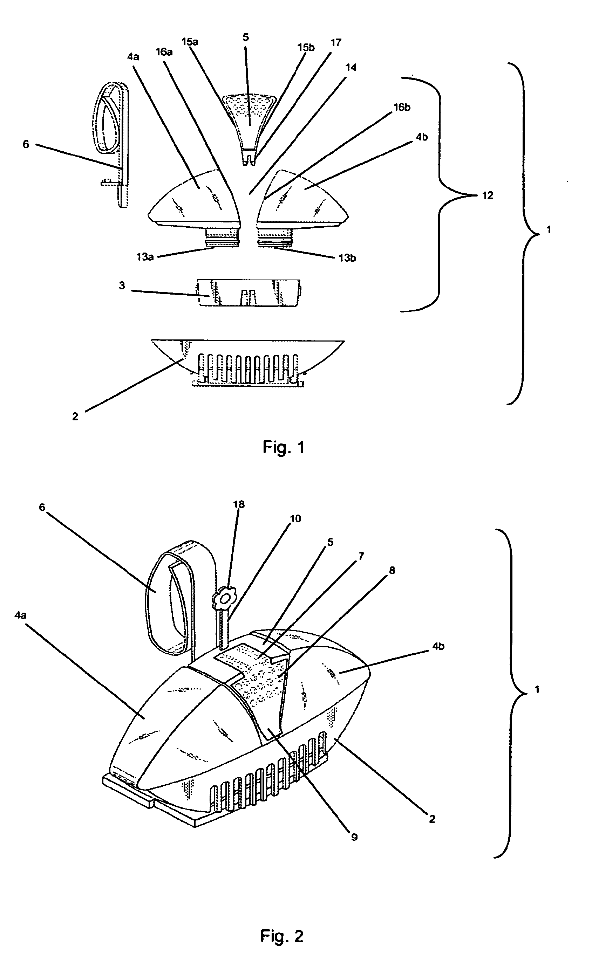

Fig. 1 zeigt eine erste Ausführungsform der Erfindung. Das WC-Körbchen 1 besteht aus einem Halter 2, in dem eine Nachfülleinheit 12 einführbar ist, die aus einem Adapter 3, in dem die beiden Vorratsbehälter 4a,4b sowie die Funktionskammer 5 fixiert sind, gebildet ist.

Die Vorratsbehälter 4a,4b sind mit dem Adapter 3 durch eine Prellverbindung verbunden, so dass die Vorratsbehälter 4a,4b üblicherweise nicht durch einen Benutzer aus dem Adapter 3 entnehmbar und flüssigkeitsdicht im Adapter 3 fixiert sind.

Im originalen, unbenutzten Zustand, sind die Auslassöffnungen 13a,13b durch eine Kappe (nicht abgebildet), die an den Vorratsbehältern 4a,4b oder am Adapter 3 ausgebildet sein kann, verschlossen, so dass ein unbeabsichtigter Austritt von Produkt aus den Vorratsbehältern 4a,4b verhindert ist. Diese Kappen werden beim Einsetzen der Nachfülleinheit 12 in den Halter 2 durch am Halter 2 ausgeformte Domen (nicht abgebildet) durchstoßen, so dass Produkt aus den Auslassöffnungen 13a,13b der Vorratsbehälter 4a,4b austreten kann.

Die Vorratsbehälter 4a,4b weisen eine in etwa dreiecksförmige Querschnittskontur auf, so dass ein ebenfalls in etwa dreiecksförmiger, keilartiger Zwischenraum 14 zwischen den Vorratsbehältern 4a,4b ausgebildet ist, wenn die Vorratsbehälter 4a, 4b im Adapter 3 fixiert sind.

In diesen Zwischenraum 14 ist eine Funktionskammer 5 einführ- und im Adapter 3 lösbar fixierbar. Die Kontur der Funktionskammer 5 entspricht im Wesentlichen der Kontur des durch die Vorratsbehälter 4a,4b gebildeten Zwischenraums 14, so dass im zusammengesteckten Zustand, die seitlichen Außenwände 15a,15b der Funktionskammer 5 an den seitlichen, den Zwischenraum 14 begrenzenden Außenwänden 16a,16b der Vorratsbehälter 4a,4b anliegen, wodurch die Funktionskammer 5 seitlich in der Nachfülleinheit 12 fixiert ist.

Am Boden der Funktionskammer 5 ist ein Bügel 17 ausgebildet, der zusammen mit einem korrespondierenden Steg (nicht abgebildet) im Adapter 3 eine lösbare Schnapp- bzw. Rastverbindung ausbildet, so dass ein unbeabsichtigtes Herausfallen der Funktionskammer 5 aus dem Adapter 3, bspw. in der ÜberKopf-Position der Nachfülleinheit 12, verhindert ist.

Am Halter 2 ist ein Bügel 6 fixierbar, durch den das WC-Körbchen 1 am Rand einer Toilette aufgehängt werden kann.
Fig. 2 zeigt eine weitere Ausführungsform des erfindungsgemäßen, bereits aus
Fig. 1 bekannten WC-Körbchens im zusammengebauten Zustand.

Die Funktionskammer 5 weist ein Griffelement 10 auf, das sich im Wesentlichen senkrecht aus der Ebene des Kopfes der Funktionskammer 5 erstreckt. Das Griffelement 10 ist stielförmig ausgebildet und weist an seinem der Funktionskammer 5 gegenüberliegendem Ende ein figuratives Element 18 auf, welches die Grifffläche für den Benutzer erhöht.

Die Funktionskammer 5 weist in ihrem Kopf Öffnungen 7 sowie in ihrer Vorderwand Öffnungen 8 auf. Durch die im Kopf der Funktionskammer 5 angeordneten Öffnungen 7 kann Spülwasser in die Funktionskammer 5 eintreten und aus den Öffnungen 8 aus der Funktionskammer 5 austreten, so dass beim Spülvorgang die Funktionskammer 5 durchspült wird.

Die Öffnungen 7,8 sind im originalen Zustand der Funktionskammer vor der ersten Verwendung der Nachfülleinheit 12 von einer abziehbaren Folie 9 verschlossen, um eine unbeabsichtigte Freisetzung von Produkt aus der Funktionskammer 5 vor Verwendung der Nachfülleinheit 12 zu verhindern.

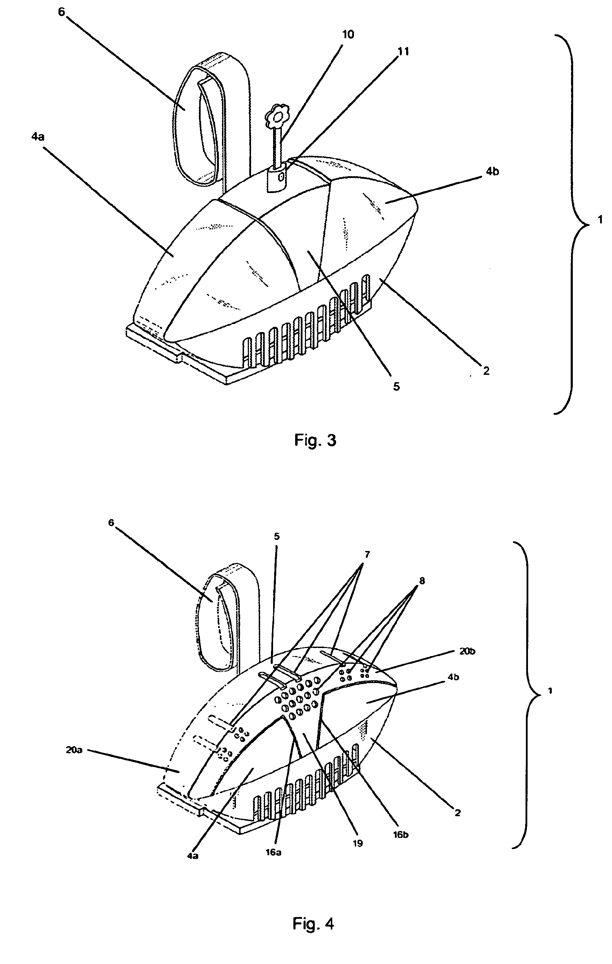

In einer weiteren Ausgestaltung kann die Funktionskammer 5, wie in Fig. 3 gezeigt, mit einem Sprühkopf 11 versehen sein. Durch Herunterdrücken des Sprühkopfes 11 wird Produkt aus der Funktionskammer 5 in den Sprühkopf 11 gefördert und durch die Düse des Sprühkopfes 11 in die Umgebung zerstäubt.

Derartige Sprühköpfe 11 sind hinlänglich aus dem Stand der Technik bekannt.

In der Fig. 3 ist die Düse des Sprühkopfes 11 im am Toilettenrand befestigten Zustand des WC-Körbchens in etwa horizontal in das Toilettenbecken hinein gerichtet. Die Düse des Sprühkopfes 11 kann jedoch auch im am Toilettenrand befestigten Zustand des WC-Körbchens schräg nach unten in die Toilettenschüssel hineingerichtet sein, um das in der Funktionskammer 5 befindliche Produkt auf die Pfanne eines Flachspülers oder den Abflussbereich eines Tiefspülers zu applizieren. Es ist jedoch auch denkbar, dass die Düse des Sprühkopfes 11 aus der Toilettenschüssel hinaus gerichtet ist.

Mit dem Sprühkopf 11 ist das Griffelement 10 verbunden, so dass beim herunterdrücken des Griffelements 10 Produkt durch den Sprühkopf 11 aus der Funktionskammer 5 abgegeben wird.

Eine weitere Ausführungsform der Funktionskammer 5 zeigt Fig. 4, in der die Funktionskammer 5 eine T-förmige Kontur ausweist. Die Funktionskammer 5 hat einen zwischen die Außenwände 16a,16b der Vorratsbehälter 4a,4b positionierbaren keilförmigen Abschnitt 19, von dem sich zu beiden Seiten flügelartige Abschnitte 20a,20b über die Kopfflächen der Vorratsbehälter 4a,4b erstrecken.

Auf der Kopffläche der Funktionskammer sind eine Mehrzahl von schlitzförmigen Öffnungen 7 angeordnet, durch die Spülwasser in die Kammer eintreten kann. In die Vorderwand der Funktionskammer 5 sind kreisförmige Öffnungen 8 eingebracht, durch die Produkt und/oder Spülwasser aus der Funktionskammer 5 austreten können.

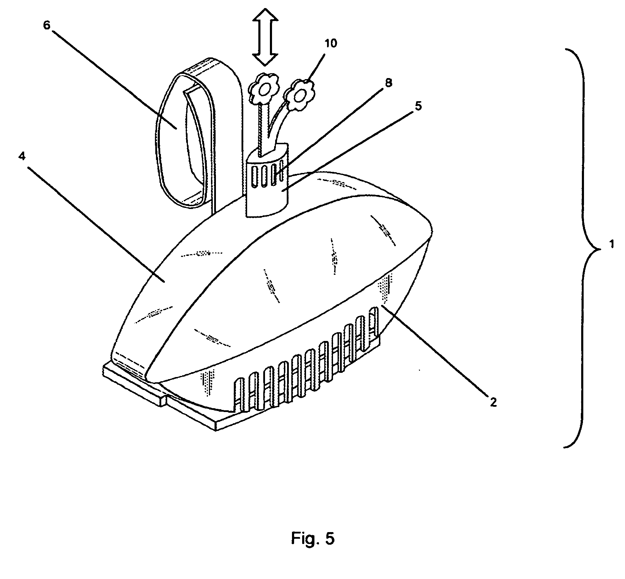

In Fig. 5 ist eine Ausgestaltung der Erfindung mit nur einem Vorratsbehälter 4 abgebildet. In oder auf der Kopffläche des Vorratsbehälters 4 ist eine Funktionskammer 5 lösbar angeordnet. Am Kopf der Funktionskammer 5 ist ein Griffelement 10 mit der Funktionskammer 5 verbunden.

Wie durch den Pfeil angedeutet, ist das Griffelement nach oben und unten bewegbar. Durch diese Bewegung des Griffelements 11 kann die Freisetzung von Aktivstoff aus der der Funktionskammer 5 gesteuert werden.

So ist es beispielsweise möglich, das Griffelement 11 mit einer in der Funktionskammer angeordneten und an der Innenwand der Funktionskammer beweglich anliegenden Hülse zu koppeln, welche in Abhängigkeit der Stellung der Hülse bzw. des Griffelements 11, die Öffnungen 8 der Funktionskammer ganz oder abschnittsweise freigibt oder vollständig verschließt.

Andererseits kann auch die Funktionskammer 5 selbst in dem Vorratsbehälter 4 verschiebbar gelagert sein, so dass beim Herausziehen der Funktionskammer 5 am Griffelement 10 die Öffnungen 8 ganz oder teilweise freigegeben werden.

## Patentansprüche

1. System (1) zum Einbringen wenigstens einer Wirkstoffsubstanz enthaiterden Zubereitung in das Spülwasser eines Toilettenbeckens umfassend
□ mindestens einen Vorratsbehälter (4) einer Nachfülleinheit (12) des Systems (1) zur Aufnahme wenigstens einer Zubereitung,
□ wobei die Nachfülleinheit (12) mit einem zur Aufnahme der Nachfülleinheit (12) vorgesehenen Halter (2) koppelbar ist und der in derart mit dem Vorratsbehälter (4) zusammenwirkt, dass Zubereitung aus dem Vorratsbehälter während eines Spülvorgangs in das Spülwasser des Toilettenbeckens freigesetzt wird,
□ wenigstens eine Funktionskammer (5), insbesondere zur Aufnahme eines Duftstoff emittierenden Trägermaterials,
□ wobei die Funktionskammer (5) lösbar mit der Nachfülleinheit (12) verbunden ist, und □ wobei an oder in der Funktionskammer(5) wenigstens ein Handhabungs- und/oder
Dosiermittel angeordnet ist,
**dadurch gekennzeichnet, dass**
□ die Funktionskammer (5) einen Sprühkopf (11) umfasst.

2. System nach Anspruch 1, **dadurch gekennzeichnet, dass** mehrere Vorratsbehälter (4a,4b) mit voneinander verschiedenen Zubereitungen vorgesehen sind.

3. System nach einem der vorherigen Ansprüche, **dadurch gekennzeichnet, dass** die Vorratsbehälter (4,4a,4b) vor dem Eintritt von Spülwasser geschützt sind.

4. System, nach Anspruch 2, **dadurch gekennzeichnet, dass** die Funktionskammer (5) zwischen zwei Vorratsbehältern (4a,4b) angeordnet ist.

5. System, nach einem der vorherigen Ansprüche, **dadurch gekennzeichnet, dass** die Funktionskammer (5) zumindest abschnittsweise von wenigstens einem Vorratsbehälter (4a,4b) umschlossen ist.

6. System, nach einem der vorherigen Ansprüche, **dadurch gekennzeichnet, dass** die Funktionskammer (5) als Prismatoid ausgeformt ist.

7. System, nach Anspruch 6, **dadurch gekennzeichnet, dass** die Funktionskammer (5) im Wesentlichen keilförmig ausgebildet ist.

8. System, nach einem der vorherigen Ansprüche, **dadurch gekennzeichnet, dass** die Funktionskammer (5) aus einem oder mehreren voneinander getrennten oder beabstandeten Räumen besteht.

9. System, nach einem der vorherigen Ansprüche, **dadurch gekennzeichnet, dass** die Verbindung zwischen der Funktionskammer (5) und der Nachfülleinheit (12) derart ausgestaltet, dass sie durch eine Zugkraft von größer als 1N von der Nachfülleinheit (12) lösbar ist.

10. System, nach Anspruch 1, **dadurch gekennzeichnet, dass** an der Funktionskammer (5) ein Griffelement (10) ausgebildet ist, durch das der Benutzer die Funktionskammer (5) in der Nachfülleinheit (12) fixiert oder aus ihr entfernt.

11. System, nach einem der vorherigen Ansprüche, **dadurch gekennzeichnet, dass** die Funktionskammer (5) mit wenigstens einer Zubereitung befüllt ist, die sich von der Zubereitung(en) in dem/den Vorratsbehälter(n) (4a,4b) unterscheidet.

12. System, nach einem der vorherigen Ansprüche, **dadurch gekennzeichnet, dass** die Funktionskammer (5) eine mechanische, elektro-mechanische und/oder elektronische Vorrichtung umschließt.

13. System nach Anspruch 12, **dadurch gekennzeichnet, dass** die Funktionskammer (5) spritzwassergeschützt ausgeführt ist.

14. System nach Anspruch 12, **dadurch gekennzeichnet, dass** die mechanische, elektromechanische und/oder elektronische Vorrichtung wenigstens eine Sensoreinheit umfasst, die physikalische, chemische und/oder mechanische Parameter aus der Umgebung der Funktionskammer bestimmen kann.

## Claims

1. A system (1) for introducing at least one preparation containing an active substance into the flush water of a toilet bowl comprising
□ at least one reservoir (4) of a refill unit (12) of the system (1) for accommodating at least one preparation,
□ the refill unit (12) being couplable with a holder (2) intended for accommodating the refill unit (12) and interacting with the reservoir (12) in such a manner that, during a flushing process, preparation is released from the reservoir into the flush water of the toilet bowl,
□ at least one functional chamber (5), in particular for accommodating a support material which emits scent,
□ the functional chamber (5) being detachably connected to the refill unit (12), and
□ at least one handling and/or dispensing means being arranged on or in the functional chamber (5),
**characterised in that**
□ the functional chamber (5) comprises a spray head (11).

2. A system according to claim 1, **characterised in that** a plurality of reservoirs (4a, 4b) with preparations which differ from one another are provided.

3. A system according to either one of the preceding claims, **characterised in that** the reservoirs (4, 4a, 4b) are protected from ingress of flush water.

4. A system, according to claim 2, **characterised in that** the functional chamber (5) is arranged between two reservoirs (4a, 4b).

5. A system according to any one of the preceding claims, **characterised in that** the functional chamber (5) is enclosed at least in places by at least one reservoir (4a, 4b).

6. A system according to any one of the preceding claims, **characterised in that** the functional chamber (5) takes the form of a prismatoid.

7. A system according to claim 6, **characterised in that** the functional chamber (5) is of substantially wedge-shaped construction

8. A system according to any one of the preceding claims, **characterised in that** the functional chamber (5) consists of one or more spaces which are separate or spaced apart from one another.

9. A system according to any one of the preceding claims, **characterised in that** the connection between the functional chamber (5) and the refill unit (12) is configured such that said functional chamber is detachable from the refill unit (12) by a tensile force of greater than 1 N.

10. A system according to claim 1, **characterised in that** a grip element (10) is provided on the functional chamber (5), by means of which grip element the user fixes the functional chamber (5) into the refill unit (12) or removes it therefrom.

11. A system according to any one of the preceding claims, **characterised in that** the functional chamber (5) is filled with at least one preparation which differs from the preparation(s) in the reservoir(s) (4a, 4b).

12. A system according to any one of the preceding claims, **characterised in that** the functional chamber (5) encloses a mechanical, electromechanical and/or electronic device.

13. A system according to claim 12, **characterised in that** the functional chamber (5) is constructed so as to be protected from water splashes.

14. A system according to claim 12, **characterised in that** the mechanical, electromechanical and/or electronic device comprises at least one sensor unit which is capable of determining physical, chemical and/or mechanical parameters from the surrounding environment of the functional chamber.

## Revendications

1. Système (1) pour l'introduction d'au moins une préparation contenant une substance active dans la chasse d'eau d'une cuvette de toilettes comprenant
o au moins un réservoir de stockage (4) d'une unité de remplissage (12) du système (1) pour la réception d'au moins une préparation,
o l'unité de remplissage (12) pouvant être couplée à un support (2) prévu pour la réception de l'unité de remplissage (12) et qui coopère avec le réservoir de stockage (12) de telle sorte que la préparation est libérée du réservoir de stockage dans l'eau de chasse de la cuvette de toilettes pendant un processus de rinçage,
o au moins une chambre fonctionnelle (5), en particulier pour la réception d'un matériau de support émettant une substance odorante,
o la chambre fonctionnelle (5) étant reliée de manière amovible à l'unité de remplissage (12), et
o sur ou dans la chambre fonctionnelle (5) au moins un moyen de manipulation et/ou de dosage étant disposé,
**caractérisé en ce que**
o la chambre fonctionnelle (5) comprend une tête de pulvérisation (11).

2. Système selon la revendication 1, **caractérisé en ce que** plusieurs réservoirs de stockage (4a, 4b) sont prévus avec des préparations différentes les unes des autres.

3. Système selon l'une des revendications précédentes, **caractérisé en ce que** les réservoirs de stockage (4, 4a, 4b) sont protégés contre la pénétration d'eau de chasse.

4. Système selon la revendication 2, **caractérisé en ce que** la chambre fonctionnelle (5) est disposée entre deux réservoirs de stockage (4a, 4b).

5. Système selon l'une des revendications précédentes, **caractérisé en ce que** la chambre fonctionnelle (5) est entourée au moins partiellement par au moins un réservoir de stockage (4a, 4b).

6. Système selon l'une des revendications précédentes, **caractérisé en ce que** la chambre fonctionnelle (5) est réalisée en forme de prismatoïde.

7. Système selon la revendication 6, **caractérisé en ce que** la chambre fonctionnelle (5) est pour l'essentiel réalisée en forme de coin.

8. Système selon l'une des revendications précédentes, **caractérisé en ce que** la chambre fonctionnelle (5) se compose d'un ou plusieurs espaces séparés ou espacés les uns des autres

9. Système selon l'une des revendications précédentes, **caractérisé en ce que** le raccord entre la chambre fonctionnelle (5) et l'unité de remplissage (12) est conçu de telle sorte qu'il est détachable de l'unité de remplissage (12) par le biais d'une force de traction supérieure à 1 N.

10. Système selon la revendication 1, **caractérisé en ce que** sur la chambre fonctionnelle (5) est réalisé un élément de prise (10) grâce auquel l'utilisateur fixe la chambre fonctionnelle (5) dans l'unité de remplissage (12) ou l'en sort.

11. Système selon l'une des revendications précédentes, **caractérisé en ce que** la chambre fonctionnelle (5) est remplie avec au moins une préparation qui est différente de la ou des préparations dans le/les réservoir(s) de stockage (4a, 4b).

12. Système selon l'une des revendications précédentes, **caractérisé en ce que** la chambre fonctionnelle (5) renferme un dispositif mécanique, électromécanique et/ou électronique.

13. Système selon la revendication 12, **caractérisé en ce que** la chambre fonctionnelle (5) est réalisée protégée contre l'eau projetée.

14. Système selon la revendication 12, **caractérisé en ce que** le dispositif mécanique, électromécanique et/ou électronique comprend au moins une unité de détection qui peut définir des paramètres physiques, chimiques et/ou mécaniques à partir de l'environnement immédiat de la chambre fonctionnelle.
